# EUROPEAN PATENT APPLICATION

(11) **EP 2 003 069 A2**
(43) Date of publication of application: **17.12.2008**
(21) Application number: 07739488.0
(22) Date of filing: 23.03.2007
(51) Int. Cl.: B65D 77/06, A61J 1/05, B65D 47/18, B65D 47/20, B65D 83/00

(54) **DISCHARGE CONTAINER AND EYE DROP CONTAINER**

(30) Priority: 28.03.2006 JP 2006089037
(71) Applicant: Otsuka Pharmaceutical Co., Ltd., Tokyo 101-8585 (JP); Otsuka Techno Corporation, Tokushima 771-0360 (JP)
(72) Inventor: SUGAHARA, Yuji, Itano-gun, Tokushima 771-0212 (JP); YAMAZAKI, Hiroyuki, Tokushima-shi, Tokushima 771-0130 (JP); ISHIKAWA, Yoshiteru, Anan-shi, Tokushima 774-0017 (JP); OGAWA, Yusuke, Anan-shi, Tokushima 779-1401 (JP); ADACHI, Shintaro, Itano-gun, Tokushima 771-0203 (JP); TOUJOU, Kousuke, Anan-shi, Tokushima 774-0021 (JP)
(74) Representative: HOFFMANN EITLE
(86) International application number: PCT/JP2007/056047
(87) International publication number: WO 2007/111256

(57) **Abstract**

A liquid delivery container comprises a bag 2 which is disposed in a container body 1 to contain a liquid and can expand and shrink or deform freely, a delivery port 3 communicating with the bag 2 and having an outlet hole 31 for discharging the liquid contained in the bag 2, a squeeze portion 6 which is disposed between the delivery port 3 and the bag 2 and is capable of deforming to discharge the liquid through the delivery port when pressed and is restorable, and the check valve 5 disposed on the upstream of the squeeze portion 6 in the discharging direction and regulating the flow of the liquid contained in the bag 2 in one way toward the squeeze portion. When the squeeze portion 6 is pressed, the liquid is discharged through the delivery port 3, and when the pressure is removed, the squeeze portion 6 restores the original shape and at the same time sucks up the liquid from the bag 2.

## Description

### Field of the Invention

The present invention relates to a liquid delivery container which contains a liquid such as eyedrop and delivers the liquid through an outlet.

### Background Art

Such containers have been used that comprise a flexible container body which contains eyedrop, liquid detergent, liquid food or other liquid, and an outlet provided on the container body for discharging the liquid contained in the container body.

In such a container, the liquid is discharged through the outlet when the container body is pressed to deform and the inner pressure of the container body becomes higher than the atmospheric pressure. Then, when the pressure applied to the container body is removed so that the inner pressure of the container body becomes lower than the atmospheric pressure, the outside air is taken in through the outlet, and the container body restores the original shape before being pressed.

However, in such a container, there is a problem that the liquid in the liquid container may be contaminated by the outside air which enters into the container since pathogenic microbe such as true fungus or virus may exist in the outside air.

To solve this problem, there has been proposed a container having such a constitution that a plug is set in a mouth of a double-structured container which is formed by laminating a peelable inner layer on the inside of an outer layer, and that an air inlet hole is formed in the outer layer so as to introduce the outside air into the interface between the inner layer and the outer layer. The plug has an outlet formed therein to discharge the liquid contained inside the inner layer, while a check valve is provided in the outlet (refer to Patent Documents 1 and 2).

With such a constitution, the liquid contained in the container body is discharged through the outlet when the container body is pressed to deform and the inner pressure of the container body becomes higher than the atmospheric pressure, and then, when the pressure applied to the container body is removed, the container body restores the original shape and the inner pressure of the container body becomes lower than the atmospheric pressure, the outside air flows into the interface between the inner layer and the outer layer.
[Patent Document 1] Japanese Unexamined Patent Publication No. 2002-80055
[Patent Document 2] Japanese Unexamined Patent Publication No. 2003-63576

### Disclosure of the Invention

### Problems to be Solved by the Invention

However, in such a double-structured container, the container requires to be pressed with a strong force in order to squeeze the bottle and increase the inner pressure sufficiently. Pressing with a light force may not be able to activate the check valve 5 provided in the outlet.

There is also a problem that the bottle requires to be pressed with even higher force when the liquid content decreases and the volume of air in the space between the inner layer and the outer layer of the double-structured bottle increases.

An object of the present invention is to provide a liquid delivery container which can sufficiently activate a pressure-operated valve installed in a delivery port even with a weak force and discharge a liquid, and an eyedrop container that uses the same.

### Means for Solving the Problems

The liquid delivery container of the present invention comprises a container body; a bag which is disposed in the container body to contain a liquid and can expand and shrink or deform freely; a delivery port communicating with the bag and having an outlet hole to discharge the liquid contained in the bag; a squeeze portion which is provided between the delivery port and the bag and is capable of deforming to discharge the liquid through the delivery port when pressed and is restorable; and a check valve provided on an upstream of the squeeze portion in a discharging direction and regulating flow of the liquid contained in the bag in one way toward the squeeze portion.

In the liquid delivery container of the present invention, the liquid can be discharged through the outlet hole simply by applying a pressure on the squeeze portion which communicates with the delivery member. Such an operation applies a pressure on the liquid which has been drawn from the upstream of the check valve or no-return valve into the squeeze portion, so that the liquid in the squeeze portion is discharged through the delivery port. When the pressure on the squeeze portion is removed, the squeeze portion is relieved of the pressing force and the squeeze portion restores the original shape, so that the inner pressure of the squeeze portion becomes lower than the atmospheric pressure and the liquid contained in the bag is drawn through the check valve into the squeeze portion. Thus the liquid delivery container has pumping function such that application of a pressure causes the liquid to be discharged through the delivery port and removing the pressure causes the restoration of the shape and the liquid to be sucked from the bag.

As a result, the liquid in the bag can be discharged by a relatively weak force of pressing the squeeze portion, regardless of the quantity of the liquid remaining in the bag.

The container body may have an air inlet hole to let air flow into a space between the container body and the bag, thereby making it easier for air to flow through the air inlet hole into a space between the container body and the bag, so that the liquid in the bag can move more easily to the squeeze portion.

The container body may have a dual structure where the container body and the bag are formed simultaneously, and the bag may have such a constitution that the inner wall of the container body can be peeled off. Such a dual structure makes it possible to manufacture the container body and the bag simultaneously, so that the manufacturing process can be simplified.

In case the container body has a pressing member provided thereon to cause elastic deformation of the squeeze portion, the user can have the liquid discharged simply by operating the pressing member.

The pressing member may be a member that is slidably disposed. The user can have the liquid discharged by a simple operation of sliding the pressing member.

The pressing member may also be a lever disposed rotatably around a pivot which is fixed on the container body.

It is preferable that a part or whole of the container body is transparent so that the bag can be observed.

The check valve has a function that the liquid contained in the bag is caused to flow into the squeeze portion when the squeeze portion is relieved of the pressure, restores the original shape and negative pressure is developed in the squeeze portion, and that the check valve closes when the inside of the container body turns to positive pressure and prevents the liquid in the squeeze portion from returning into the bag. This function of the check valve enables to discharge the liquid in the squeeze portion through the delivery port.

The check valve may include a float which moves up and down depending on the pressure difference between the squeeze portion and the bag.

The squeeze portion is preferably formed from a flexible and pliant material, such as silicone rubber, vinyl chloride, polyethylene, or polypropylene, or a composite of these materials.

The delivery port preferably has the function of so-called pressure-operated valve which closes the outlet hole when no liquid pressure is applied from the upstream in the delivering direction, and discharges the liquid through the outlet hole when liquid pressure is applied from the upstream in the delivering direction. With this function, when the squeeze portion is relieved of the pressing force and restores the original shape and the inner pressure of the squeeze portion becomes lower than the atmospheric pressure, the outlet hole is immediately closed, thereby making it possible to prevent the outside air and the liquid remaining around the outlet hole from flowing into the squeeze portion.

The delivery port may have a constitution that comprises a cover having an outlet hole formed therein, a valve element support disposed in the cover and having a distal end shaft thereof exposed through the outlet hole formed in the cover, and a valve element which surrounds the distal end shaft of the valve element support. When no liquid pressure is applied from the squeeze portion, the valve element is in contact with the distal end shaft so as to close the outlet hole, and when a liquid pressure is applied from the squeeze portion, the valve element deforms to form a flow passage between the valve element and the distal end shaft, thereby opening the outlet hole. As the outlet hole is opened, the outlet hole communicates with the portion on the upstream of the delivery port (the squeeze portion communicating with the delivery member), thereby achieving the discharge of the liquid through the outlet hole.

Such a liquid delivery container is preferably applied to, for example, an eyedrop container which uses a squeeze bottle.

According to the present invention, as described above, since the volume of liquid contained in the squeeze portion remains substantially constant even when the liquid remaining in the bag decreases, the liquid can be discharged through the delivery port with a constant force. It also eliminates the need to use an air filter which may be blocked.

The above and other advantages, features and effects of the present invention will become apparent through the following description of embodiments with reference to the accompanying drawings.

### Brief Description of the Drawings

Fig. 1 is a side sectional view of an eyedrop container according to an embodiment of the present invention.
Fig. 2 is an exploded perspective view of the eyedrop container.
Fig. 3(a) is a sectional view explaining a process of applying eyedrops.
Fig. 3(b) is a sectional view explaining the process of applying eyedrops.
Fig. 3(c) is a sectional view explaining the process of applying eyedrops.
Fig. 4 is a perspective view showing the configuration of a check valve 5.
Fig. 5 is a side sectional view of an eyedrop container according to another embodiment of the present invention.
Fig. 6 is a side sectional view of an eyedrop container according to still another embodiment of the present invention.
Fig. 7 is an exploded perspective view showing the relationship between a float 82 and a float holder 83.
Fig. 8 is diagram showing the state of a check valve when the liquid flow is stopped.
Fig. 9 is diagram showing the state of the check valve when the liquid is allowed to flow.
Fig. 10 is a sectional view showing another configuration of a pressing member which presses the side surface of a squeeze portion 6.
Fig. 11 is a sectional view showing the configuration of another check valve.

### Description of Reference Numerals

- 1: Container body
- 1a: Air inlet hole
- 2: Bag
- 3: Delivery port
- 4: Inner cylindrical portion
- 5, 5A, 5B: Check valve
- 6: Squeeze portion
- 7: Pressing member
- 31: Outlet hole
- 32: Cover
- 33: Distal end shaft
- 34: Valve element support
- 35: Second nozzle
- 36: Cylindrical member
- 37: Valve element
- 41: Opening
- 42: Inward protruding portion
- 43: Flange
- 44: Cylindrical member
- 45: First nozzle
- 51: Distal end portion
- 52: Bottom portion
- 71: Groove
- 82: Float

### Best Mode for Carrying Out the Invention

Embodiments of the present invention will now be described in detail with reference to the accompanying drawings.

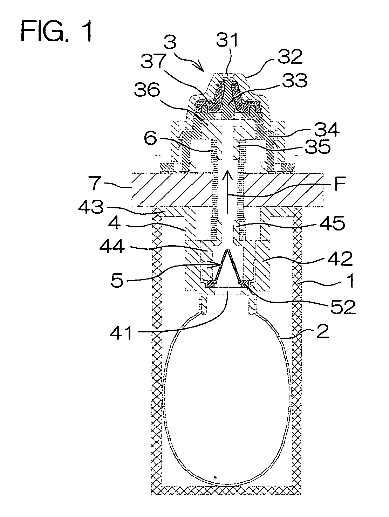

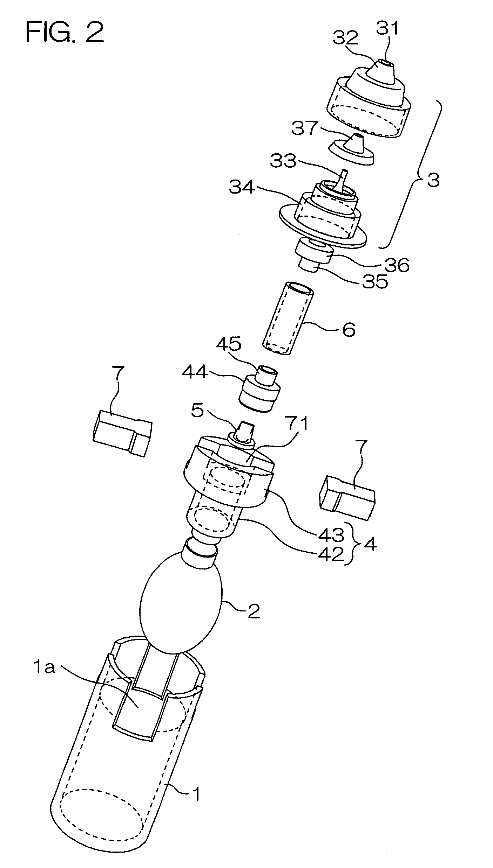

Fig. 1 is a side sectional view of an eyedrop container according to an embodiment of the present invention, and Fig. 2 is an exploded perspective view thereof.

The eyedrop container comprises a container body 1, a bag 2 disposed in the container body 1 to contain eyedrop liquid, and a delivery port 3 which communicates with the bag 2 and opens when the inner pressure of a squeeze portion 6 becomes higher than the atmospheric pressure so as to discharge the liquid contained in the squeeze portion 6.

The container body 1 is a container for containing the bag 2. The container is made of a transparent resin at least in a portion where the bag is seen. Transparency is required in order to visually check the quantity of the liquid in the bag 2. The container body 1 is also preferably made of a hard material so as to ensure high durability, since it is not intended to deform the container body 1 itself by depressing it with hand.

The delivery port 3 and the container body 1 of the present invention may be formed from a resin such as polyethylene (PE), polypropylene (PP) or cyclo-olefin polymer (COP). In particular, PE and PP are safe from the medical viewpoint and are preferably used for the liquid delivery container of the present invention when the container is used as a container containing a medical fluid such as eyedrop container.

An inner cylindrical portion 4 is inserted into an opening located at the top of the container body 1. The bag 2 is mounted on an opening 41 which is located at the bottom of the inner cylindrical portion 4 and faces the inside of the container body 1.

The bag 2 is formed from a material which can freely deform and contains a liquid such an eyedrop, for example, a pliable single-layer or multi-layer film of polyolefin (PE, PP, etc.), a single-layer or multi-layer film of polyester (PET, etc.) or a composite multi-layer film combining them.

A constitution in which air can be introduced through an air inlet hole 1a (refer to Fig. 2) formed in the container body 1 into the interface between the bag 2 and the container body 1 is employed.

The inner cylindrical portion 4 comprises an inward protruding portion 42 having cylindrical shape of a relatively small diameter and inserted into the container body 1, and a flange 43 having a relatively large diameter and bonded onto the end face of the opening of the container body 1 when inserted into the container body 1.

A check valve 5 is installed in the opening of the inward protruding portion 42 at the inner bottom surface thereof, for flowing the liquid contained in the bag 2 downstream in the discharging direction (upward direction F in Fig. 1).

A cylindrical member 44, having a first nozzle 45 for mounting the tube-shaped squeeze portion 6 which can be deformed and restore the original shape when pressed so as to discharge the liquid contained in the bag 2, is disposed above the check valve 5 in the inward protruding portion 42, in such a posture where the first nozzle 45 faces upward. The opening of the squeeze portion 6 located at the lower end thereof is mounted to the first nozzle 45. Bottom surface of the cylindrical member 44 is in close contact with the top surface of a bottom 52 of the check valve 5.

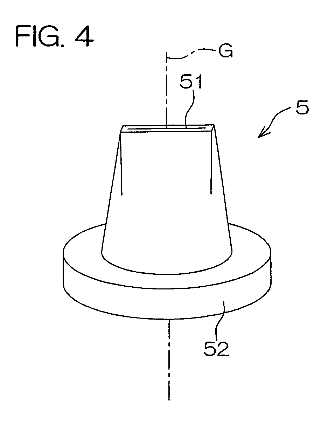

The check valve 5 is formed in a cylindrical shape from a flexible material, with a distal end portion 51 thereof being cut perpendicularly to center axis G so as to have an elongated shape with both ends thereof being fused, as shown in Fig. 4. The bottom 52 retains round shape.

The distal end portion 51 of the check valve 5 is required to be easily deformed by the liquid pressure which is applied when the squeeze portion is pressed, so as to open when the surrounding pressure becomes negative and not to open when the surrounding pressure becomes positive. For this reason, the check valve is formed from a highly pliant material having low hardness. For example, the check valve 5 is formed from a rubber-like material such as thermoplastic elastomer, natural rubber, silicone rubber, isoprene rubber, butyl rubber, butadiene rubber or fluorine-containing rubber.

The delivery port 3 comprises a generally cylindrical cover 32 having an outlet hole 31 formed at the distal end thereof and opened upward, a generally cylindrical valve element support 34 disposed in the cover 32 and having a distal end shaft 33 thereof being exposed through the outlet hole 31 formed in the cover 32, and a valve element 37 which surrounds the distal end shaft 33 of the valve element support 34. The distal end shaft 33 has a solid structure.

The outlet hole 31, the valve element 37 and the valve element support 34 of the delivery port 3 constitute a pressure-operated valve having back flow preventing function that prevents the liquid and air from flowing back.

Normally the valve element 37 makes contact with the valve element support 34 and closes the outlet hole 31. When the pressure in the squeeze portion 6 becomes higher than the atmospheric pressure during use of the eyedrop container, a space is formed between the distal end shaft 33 and the valve element 37 that surrounds the distal end shaft 33, thereby discharging the liquid contained in the squeeze portion 6 through the outlet hole 31 to the outside of the container body 1.

When the pressure in the squeeze portion 6 becomes lower than the atmospheric pressure, the valve element 37 quickly restores the original shape, so that the valve element 37 makes contact with the distal end shaft 33 of the valve element support 34 and blocks the flow passage formed in the outlet hole 31. Thus the liquid and air are prevented from entering the squeeze portion 6.

The valve element 37 is required to be easily deformed by the liquid pressure when the squeeze portion is pressed. Therefore, the valve element 37 is required to be formed from a highly pliant material having low hardness, similarly to the check valve 5.

The bottom surface of the valve element support 34 makes contact with the top surface of the flange 43. A cylindrical member 36 having a second nozzle 35 for mounting the top end of the squeeze portion 6 is disposed in the valve element support 34, in such a state that the second nozzle 35 thereof faces downward.

The first nozzle 45 and the second nozzle 35 secure the bottom end and top end of the squeeze portion 6, respectively. While there is no restriction on the method of securing, for example, elastic force of the squeeze portion 6, an adhesive or laser fusing may be employed.

The flange 43 has a groove 71 formed in a direction toward the center (the squeeze portion 6) for inserting two pressing members 7 which press the side surfaces of the squeeze portion 6.

The pressing member 7 has, for example, shape of rectangular parallelepiped, and is disposed slidably with respect to the groove 71.

The squeeze portion 6 has flexibility so as to deform and decrease the inner volume thereof when pressed by the pressing member 7 sideways, and to restore the original shape and volume when the pressure is removed. For this reason, the squeeze portion 6 is formed from a material which has high elasticity and flexibility.

For example, the squeeze portion 6 may be formed from an elastic material such as silicone rubber, vinyl chloride, polyethylene, or polypropylene, or a composite of these materials.

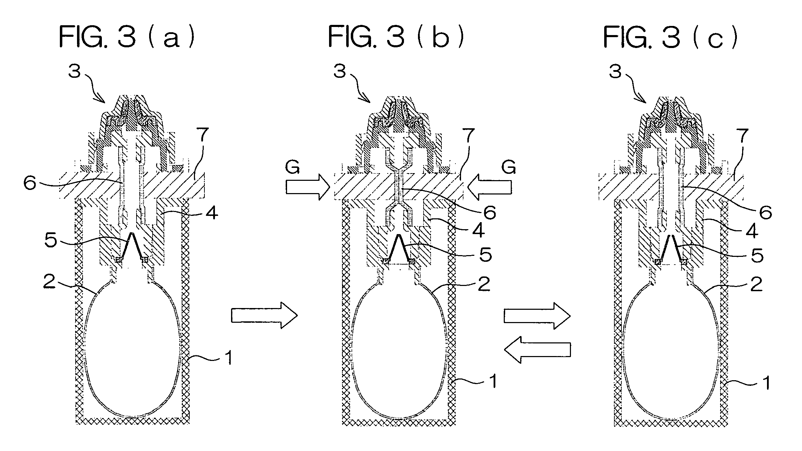

The operation of applying eyedrops will now be described with reference to Fig. 3(a) through Fig. 3(c).

When the pressing members 7 are not operated, the pressing members 7 are kept at the outward positions by the elastic force of the squeeze portion 6 which is not deformed, as shown in Fig. 3(a). Inner pressure of the squeeze portion 6 and the liquid pressure in the bag 2 are equal to each other, and the check valve 5 is closed.

When the pressing members 7 are pressed, the squeeze portion 6 is pressed by the pressing members 7 on both sides and the inner volume of the squeeze portion 6 decreases, as shown in Fig. 3(b). As a result, inner pressure of the squeeze portion 6 increases, so that the liquid in the squeeze portion 6 is discharged through the delivery port 3. At this time, since the check valve 5 closes, the liquid in the squeeze portion 6 is not capable of returning into the bag 2.

When the pressure on the pressing members 7 is removed, the squeeze portion 6 restores the original shape as shown in Fig. 3(c), and the pressure in the squeeze portion 6 turns to negative. Then, the liquid contained in the bag 2 flows through the check valve 5 and enters in the squeeze portion 6. The back flow preventing mechanism of the delivery port 3 prevents the outside air and liquid from flowing through the outlet hole 31 into the squeeze portion 6.

When the pressing members 7 are pressed again, the squeeze portion 6 is pressed by the pressing members 7 on both sides and the liquid in the squeeze portion 6 is discharged through the delivery port 3. When the pressure on the pressing members 7 is removed again, the liquid contained in the bag 2 flows through the check valve 5 and fills the squeeze portion 6. This process is repeated.

As use of the eyedrop container is continued, the liquid filling the bag 2 decreases and accordingly the bag 2 shrinks. As the container body 1 is formed from a transparent resin, the user can always monitor the state of the bag 2.

The constitution of the eyedrop container described above makes it possible to discharge the liquid contained in the bag with a relatively weak force of pressing the squeeze portion, regardless of the quantity of the liquid remaining in the bag 2.

The delivery port 3 of the present invention can be used as a liquid filter. The liquid filter allows the liquid to pass when a pressure above a threshold is applied. Providing such a filter can prevent germ, dust and other matter from entering (flowing back) into the container.

Another embodiment of the present invention will now be described.

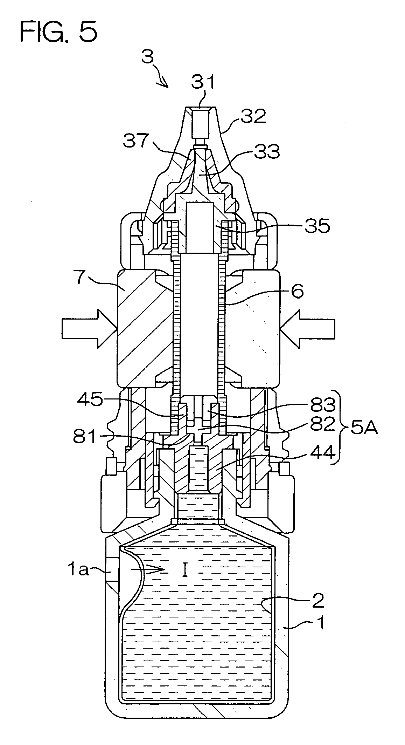

Fig. 5 is a side sectional view of an eyedrop container according to this embodiment.

Difference of this eyedrop container from the eyedrop container shown in Fig. 1 will be described below. In the eyedrop container, the container body 1 and the bag 2 installed on the inner wall of the container body 1 are formed at the same time, so as to form a double-structured container consisting of the container body 1 and the bag 2. However, the container body 1 and the bag 2 are not bonded to each other, and the bag 2 can be peeled off from the inner surface of the container body 1. The container body 1 has, on a predetermined position on the side surface thereof, the air inlet hole 1a formed in the container body 1 while the bag 2 is not broken.

As the liquid is discharged, the bag 2 peels off from the inner wall of the container body 1 near the air inlet hole 1a as indicated by arrow I and the peeling expands thereafter, so that the bag is folded up inward. The air inlet hole 1a may be formed at any position, not limited in the side surface of the container body 1 and may be formed, for example, in the bottom surface.

The container body 1 may be formed from a material such as polyethylene (PE), polypropylene (PP), cyclo-olefin polymer (COP) or polyester (PET). The bag 2 is formed from a material, for example, a pliable single-layer or multi-layer film of polyolefin (PE, PP, etc.), a single-layer or multi-layer film of polyester (PET, etc.) or a composite multi-layer film combining them.

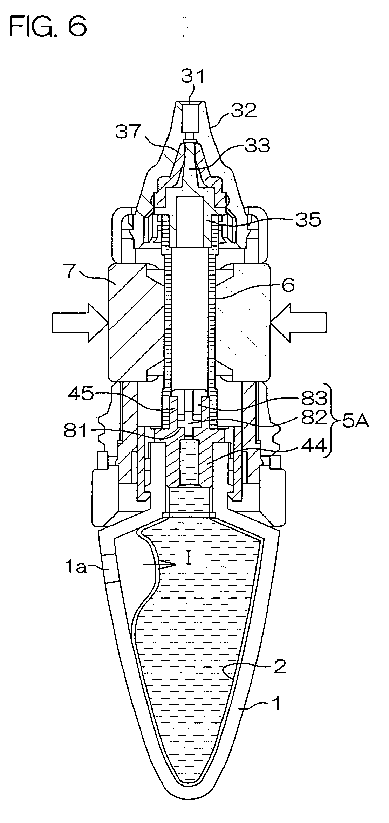

Fig. 6 is a side sectional view of an eyedrop container according to still another embodiment. This eyedrop container is different from the eyedrop container shown in Fig. 5 only in the shape of the container body 1. While the container body 1 of the eyedrop container shown in Fig. 5 has cylindrical shape, the container body 1 of the eyedrop container shown in Fig. 6 has conical shape. It is considered that the container body 1 having conical shape makes it easier for the bag 2 to peel off.

The eyedrop containers shown in Fig. 5 and Fig. 6 are different in the shape and structure of a check valve from those of the check valve shown in Fig. 1.

That is, the check valve 5A of this embodiment has a structure in which the cylindrical member 44 having the first nozzle 45 for mounting the tube-shaped squeeze portion 6 has the inner diameter reduced stepwise at a mid position, and a valve seat 81 is formed at the position.

A float 82 is placed on the valve seat 81, and a float holder 83 is placed over the float 82. The float 82 is preferably formed from a synthetic resin having a value of specific gravity that is lower than to that of the liquid.

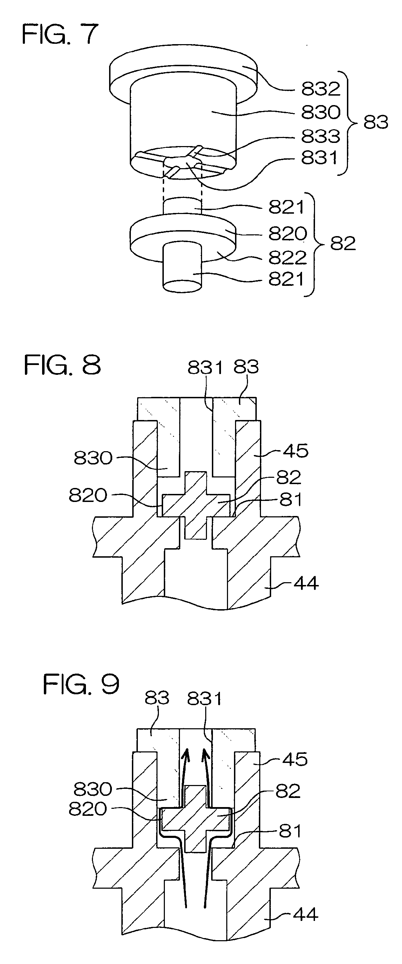

Fig. 7 is an exploded perspective view showing the relationship between the float 82 and the float holder 83. The float 82 has rotationally symmetric shape, and comprises a columnar member 821 and a disk 820 having a diameter larger than that of the columnar member 821 formed at the center of the columnar member 821. The outer diameter of the disk 820 is smaller than the inner diameter of the first nozzle 45. The float holder 83 has columnar shape and has a hole 831 of cylindrical shape for inserting the cylinder 821 therein. The hole 831 and the valve seat 81 have inner diameters larger than the outer diameter of the columnar member 821. A flange 832 is formed at the top end of the columnar member 830 for holding the float holder 83 onto the first nozzle 45 of the cylindrical member 44. The bottom surface of the columnar member 830 further has a liquid communication passage 833 formed in cross-shaped groove for supplying the liquid contained in the bag 2 to the squeeze portion 6.

The operation of the check valve 5A will now be described below with reference to Fig. 8 and Fig. 9. Fig. 8 shows the state of the float 82 when the liquid flow is stopped. Fig. 9 shows the state of the float 82 when the liquid flows.

When the inner pressure of the squeeze portion 6 is made higher than the inner pressure of the bag 2 by pressing the side surface of the squeeze portion 6, the float 82 moves down (Fig. 8). In this state, the bottom surface of the disk 820 of the float 82 makes close contact with the valve seat 81 of the cylindrical member 44, and the liquid cannot pass between the squeeze portion 6 and bag 2.

When the inner pressure of the squeeze portion 6 is made lower than the inner pressure of the bag 2 by removing the pressure from the side surface of the squeeze portion 6, the float 82 moves up (Fig. 9). At this time, while the top surface of the disk 820 makes contact with the bottom surface of the columnar member 830, the liquid contained in the bag 2 passes through the side wall of the first nozzle 45, the communication passage 833 and the hole 831 and flows into the squeeze portion 6, since the communication passage 833 is provided.

In this way, the operation of the check valve 5A can be achieved by squeezing the squeeze portion 6 so as to move the float 82 up and down. Thus the liquid contained in the bag can be discharged through the delivery port 3 with a relatively light force of simply depressing the squeeze portion.

The embodiments of the present invention have been described, but the present invention is not limited to these embodiments, and various modifications can be made within the scope of the present invention.

For example, the pressing member to press the side surface of the squeeze portion 6 is not limited to one which can slide in one direction as shown in Fig. 1 and Fig. 2. For example, it may be a lever which can rotate around a pivot.

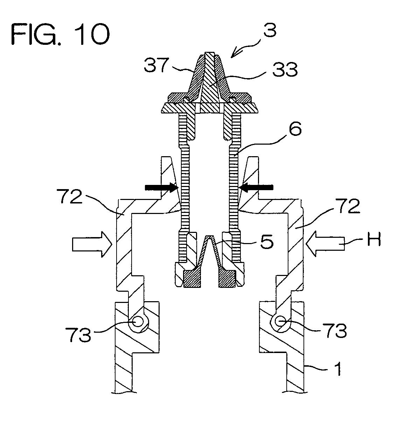

Fig. 10 is a sectional view of an eyedrop container having a pivot 73 fixed at a predetermined position of the container body 1 instead of the slidable pressing member 7, and an end of pressing lever 72 is pivotably attached to the pivot 73. The cover 32 and the bag 2 are omitted in this drawing. When the pressing lever 72 is pressed in as indicated by arrow H, the other end of the pressing lever 72 is pressed against the squeeze portion 6, thereby deforming it.

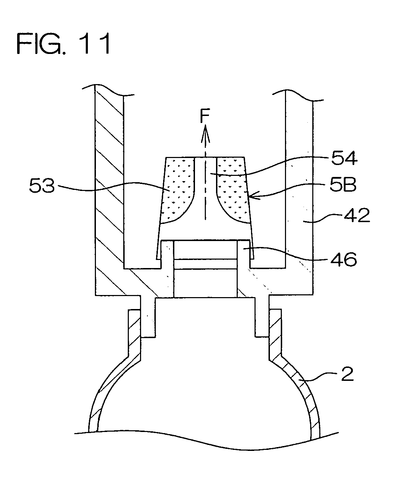

The shape and structure of the check valve 5 are not also limited to those described above. For example, as shown in Fig. 11, a cylindrical rib 46 may be formed to erect upward around the opening 41 of the cylindrical inward protruding portion 42, and a check valve 5B may be mounted on the rib 46. The check valve 5B may be mounted by fusing two flexible films having elongated shape to each other on both side portions 53 thereof, disposing the lower sides of the films closely so as to wind around the rib 46 and thermally fusing them. Thus an intra-valve passage 54 can be formed in the film. The film may be a film formed of polyolefin (PE, PP, etc.) or polyester (PET, etc.) or a composite multi-layer film combining them. When the liquid flows from the bag 2 in the upward direction F in Fig. 11, the intra-valve passage 54 opens and the flow of the liquid is not blocked, while the films flex to contact each other and block the entry of the liquid, when the liquid flows from the upper side to the intra-valve passage 54.

The liquid delivery container of the present invention can be used, in addition to the eyedrop container, for containers for medical fluids such as nose drops, ear drops, mouthwash and externally applied medicines, cosmetics container, disinfectant container, liquid detergent container and the like.

## Claims

1. A liquid delivery container comprising:
a container body;
a bag which is disposed in the container body to contain a liquid and can expand and shrink or deform freely;
a delivery port communicated with the bag and having an outlet hole to discharge the liquid contained in the bag;
a squeeze portion which is provided between the delivery port and the bag and is capable of deforming to discharge the liquid through the delivery port when pressed and is restorable; and
a check valve provided on an upstream of the squeeze portion in a discharging direction and regulating flow of the liquid contained in the bag in one way toward the squeeze portion.

2. The liquid delivery container according to claim 1, wherein the container body has a dual structure where the container body and the bag are formed simultaneously, and where the bag can be peeled off from an inner wall of the container body.

3. The liquid delivery container according to claim 2, wherein the container body has an air inlet hole to let air flow into a space between the container body and the bag.

4. The liquid delivery container according to claim 1, wherein the container body has a pressing member to cause elastic deformation of the squeeze portion.

5. The liquid delivery container according to claim 4, wherein the pressing member is slidably disposed toward the squeeze portion.

6. The liquid delivery container according to claim 5, wherein the pressing member includes a lever disposed pivotably around a pivot which is fixed on the container body.

7. The liquid delivery container according to claim 1, wherein a part or whole of the container body is transparent.

8. The liquid delivery container according to claim 1, wherein the check valve allows the liquid contained in the bag to flow into the squeeze portion when the squeeze portion is relieved of a pressure and restores an original shape and a negative pressure as compared with that of the bag is developed in the squeeze portion, and the check valve closes and prevents the liquid in the squeeze portion from returning into the bag when the inside of the container body turns to a positive pressure as compared with that of the bag.

9. The liquid delivery container according to claim 8, wherein the check valve includes a float which moves up and down depending on a pressure difference between the squeeze portion and the bag.

10. The liquid delivery container according to claim 1, wherein the squeeze portion is formed from a flexible and pliant material.

11. The liquid delivery container according to claim 10, wherein the squeeze portion is formed of silicone rubber, vinyl chloride, polyethylene, or polypropylene, or a composite of these materials.

12. The liquid delivery container according to claim 1, wherein the delivery port closes the outlet hole when no liquid pressure is applied from the upstream in the discharging direction, and discharges the liquid through the outlet hole when a liquid pressure is applied from the upstream in the discharging direction.

13. The liquid delivery container according to claim 12, wherein the delivery port comprises a cover having an outlet hole formed therein, a valve element support disposed in the cover and having a distal end shaft exposed through the outlet hole formed in the cover and a valve element which surrounds the distal end shaft of the valve element support, and the valve element is in contact with the distal end shaft so as to close the outlet hole when no liquid pressure is applied from the squeeze portion, and the valve element deforms to form a flow passage between the valve element and the distal end shaft and open the outlet hole when a liquid pressure is applied from the squeeze portion.

14. An eyedrop container which uses the container of claim 1 for administering eyedrops.
